Europäisches Patentamt

European Patent Office

Office européen des brevets

(11) Veröffentlichungsnummer: **0 393 214**

**A1**

(12) # EUROPÄISCHE PATENTANMELDUNG

(21) Anmeldenummer: **89106847.0**

(22) Anmeldetag: **17.04.89**

(51) Int. Cl.5: **A61B 6/10, G21F 3/00, G21F 7/02**

(43) Veröffentlichungstag der Anmeldung:
**24.10.90 Patentblatt 90/43**

(84) Benannte Vertragsstaaten:
**DE FR**

(71) Anmelder: **Siemens Aktiengesellschaft**
**Wittelsbacherplatz 2**
**D-8000 München 2(DE)**

(72) Erfinder: **Pfeiler, Manfred, Dr.-Ing.**
**Ludwig-Thoma-Strasse 9**
**D-8520 Erlangen(DE)**
Erfinder: **Seyler, Gerhard, Dipl.-Ing. (FH)**
**Lukasstrasse 6**
**D-8526 Bubenreuth(DE)**

(54) **Röntgendiagnostikgerät mit einer Strahlenschutzvorrichtung.**

(57) Es soll ein Röntgendiagnostikgerät mit flexiblen Streifen (8) aus strahlenabsorbierendem Material so ausgebildet werden, daß bei gutem Strahlenschutz und guter Zugänglichkeit zum Patienten eine einwandfreie Sichtkontrolle des untersuchten Bereiches gewährleistet ist.

Die Streifen (8) bestehen aus gelenkig miteinander verbundenen Bleiglasscheiben (9, 10, 11). Die Scharniere zwischen den Bleiglasscheiben (9, 10, 11) sind vorzugsweise ebenfalls aus strahlenabsorbierendem Material hergestellt.

FIG 1

Die Erfindung betrifft ein Röntgendiagnostikgerät mit flexiblen Streifen aus strahlenabsorbierendem Material zum Strahlenschutz.

Es ist ein Röntgendiagnostikgerät dieser Art bekannt, bei dem zum Schutz des Untersuchers gegenüber austretender Strahlung im Bereich der Patientenliege schwenkbare Bleigummilappen vorgesehen sind. Diese Bleigummilappen gewährleisten zwar einen guten Strahlenschutz und eine gute Zugänglichkeit zum Patienten, erlauben jedoch, da sie einen Teil des Patienten verdecken, nur schwer eine Sichtkontrolle. Dies stört insbesondere bei interventionellen Techniken.

Der Erfindung liegt demgemäß die Aufgabe zugrunde, ein Röntgendiagnostikgerät der eingangs genannten Art so auszubilden, daß bei gutem Strahlenschutz und guter Zugänglichkeit zum Patienten für den Untersucher auch ein guter Sichtkontakt zwischen Untersucher und Patient gewährleistet ist.

Diese Aufgabe ist erfindungsgemäß dadurch gelöst, daß die Streifen aus gelenkig miteinander verbundenen Bleiglasscheiben bestehen. Die Bleiglasscheiben können dabei in ihrer Größe den Forderungen an die Flexibilität angepaßt werden. Sie können ganz oder teilweise weggeklappt werden, so daß der Patient gut zugänglich ist oder klappen sich aus der normalerweise vertikalen Lage heraus, wenn aus Gründen der Aufnahmegeometrie bzw. Projektionsrichtung die Bleiglasscheiben mit Patient, Tisch oder Zubehör in Berührung kommen. In jedem Fall ist eine einwandfreie Sichtkontrolle möglich.

Eine Weiterbildung der Erfindung besteht darin, daß auch die Scharniere zwischen den Bleiglasscheiben aus strahlenabsorbie rendem Material bestehen. Dadurch ist verhindert, daß an den Verbindungsstellen der Bleiglasscheiben unzulässige Strahlung austritt.

Die Erfindung ist nachfolgend anhand zweier in den Zeichnungen dargestellter Ausführungsbeispiele näher erläutert. Es zeigen:

Fig. 1 und 2 zwei Varianten eines Röntgendiagnostikgerätes gemäß der Erfindung, und

Fig. 3 und 4 Einzelheiten des Röntgendiagnostikgerätes gemäß den Fig. 1 und 2.

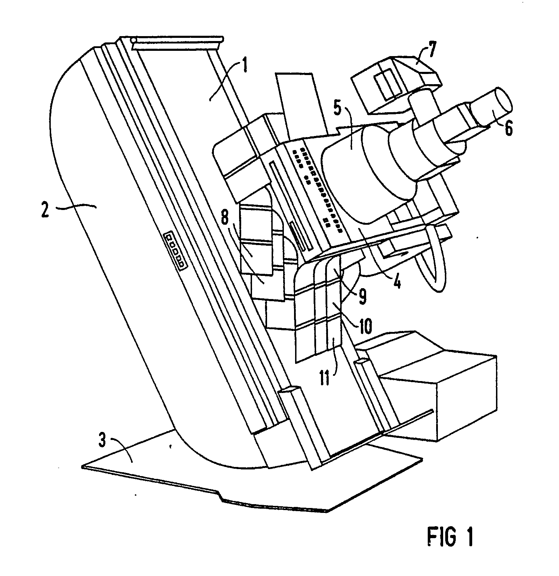

In der Fig. 1 ist ein kippbares Röntgendiagnostikgerät mit einer Patientenliege 1 an einem Rahmen 2 dargestellt. Der Rahmen 2 ist auf einem Sockel 3 kippbar gelagert. Der Röntgenstrahler ist im Rahmen 2 in nicht sichtbarer Weise angeordnet. Die Bilderzeugung erfolgt mit Hilfe eines Röntgenzielgerätes 4, eines Röntgenbildverstärkers 5 und diesem nachgeschalteten Bildaufnahmesystemen 6, 7.

Zum Schutz des Untersuchers gegen seitlich austretende Strahlung ist am Rahmen des Zielgerätes 4 eine Anzahl von Streifen 8 vorgesehen.

Jeder der Streifen 8 ist von einer Reihe von Bleiglasscheiben 9, 10, 11, z.B. auch aus Kunstglas, gebildet, welche gelenkig miteinander verbunden sind. Die Bleiglasscheiben 9, 10, 11 können demgemäß ganz oder teilweise weggeklappt werden, damit der Untersucher freien Zugang zum Patienten auf der Patientenliege 1 hat. Sie richten sich der Schwerkraft entsprechend aus und überlappen einander in jeder Kippstellung des Röntgendiagnostikgerätes. Sie erlauben eine gute Beobachtung des untersuchten Bereiches des Patienten.

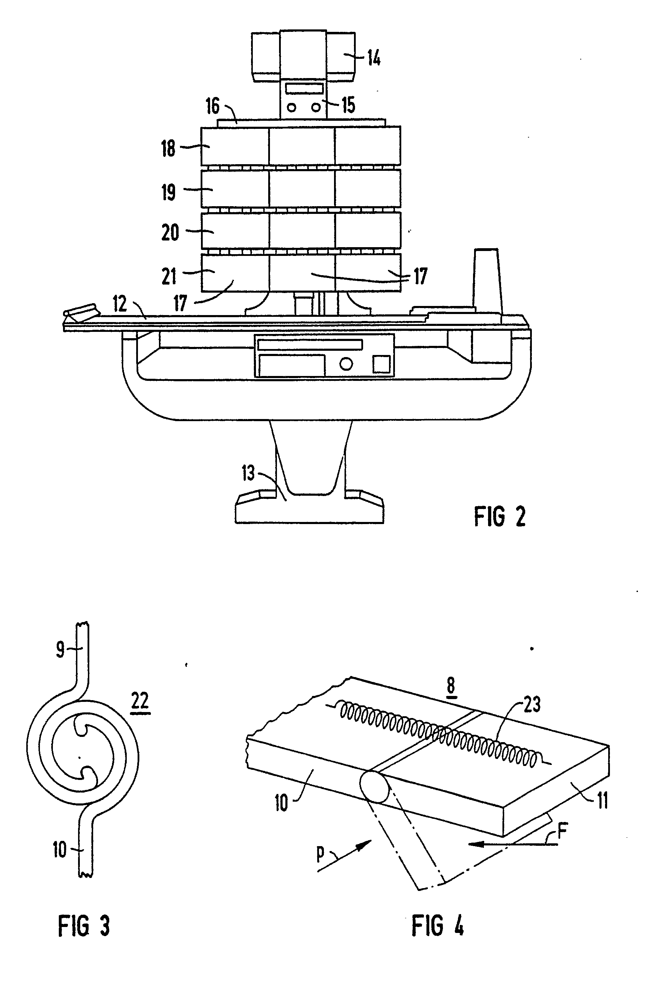

In der Fig. 2 ist ein Röntgendiagnostikgerät mit einer Patientenliege 12 dargestellt, welche auf einem Sockel 13 gelagert ist. Der Röntgenstrahler 14 liegt über der Patientenliege 12. Im Bereich seiner Primärstrahlenblende 15 ist eine Halterung 16 für Streifen 17 vorgesehen. Jeder der Streifen 17 besteht wieder aus einer Reihe aufeinanderfolgender Bleiglasscheiben 18, 19, 20, 21, die gelenkig miteinander verbunden sind.

Bei beiden Ausführungsbeispielen bestehen die Scharniere zwischen den Bleiglasscheiben 9, 10, 11 bzw. 18 bis 21 vorzugsweise ebenfalls aus strahlenabsorbierendem Material, so daß ein sicherer Strahlenschutz auch in den Übergangsbereichen zwischen jeweils zwei Bleiglasscheiben 9, 10, 11, 18 bis 21 gewährleistet ist.

Die Fig. 3 zeigt als Beispiel die Verbindung der Bleiglasscheiben 9, 10 durch ein Röntgenstrahlen absorbierendes Scharnier 22, wie es auch zwischen den anderen Bleiglasscheiben vorgesehen ist. Das Scharnier 22 ist von den beiden durch Zusammendrücken ineinander geschnappten gleichartigen, hakenförmig geformten Enden der Bleiglasscheiben 9, 10 gebildet.

Die Fig. 4 zeigt als Beispiel die Bleiglasscheiben 10, 11 eines Streifens 8. Dieser weist insgesamt eine von der Vertikalen abweichende Vorzugsrichtung unter Beibehaltung seiner Grundform auf. Wirkt eine Kraft F auf die Bleiglasscheibe 11, so klappt diese entgegen der Kraft einer Feder in die gestrichelt gezeichnete Stellung. Normalerweise hält die Feder 23 den Streifen 8 gestreckt in der Vorzugsrichtung. Entsprechend können auch die anderen Streifen ausgebildet sein.

**Ansprüche**

1. Röntgendiagnostikgerät mit flexiblen Streifen (8, 17) aus strahlenabsorbierendem Material zum Strahlenschutz, **dadurch gekennzeichnet,** daß die Streifen (8, 17) aus gelenkig miteinander verbundenen Bleiglasscheiben (9, 10, 11, 18 bis 21) bestehen.

2. Röntgendiagnostikgerät nach Anspruch 1, **dadurch gekennzeichnet,** daß die Scharniere zwischen den Bleiglasscheiben (9, 10, 11, 18 bis 21) ebenfalls aus strahlenabsorbierendem Material bestehen.

3. Röntgendiagnostikgerät nach Anspruch 2, **dadurch gekennzeichnet,** daß die Scharniere (22) an den Enden der Bleiglasscheiben (9, 10, 11, 18 bis 21) angeformt sind und sich die Scharnierfunktion durch Zusammendrücken zweier gleichartiger Bleiglasscheibenenden bis zum Einschnappen ergibt.

4. Röntgendiagnostikgerät nach einem der Ansprüche 1 bis 3, **dadurch gekennzeichnet,** daß jeder Streifen (8, 17) aus einer Reihe von aufeinanderfolgenden Bleiglasscheiben (9, 10, 11, 18 bis 21) besteht.

5. Röntgendiagnostikgerät nach einem der Ansprüche 1 bis 4, **dadurch gekennzeichnet,** daß mindestens ein Streifen (8, 17) insgesamt eine von der Vertikalen abweichende Vorzugsrichtung (unter Beibehaltung seiner Grundform) aufweist, wobei der Streifen (8, 17) durch eine Federanordnung (23) gestreckt gehalten wird, bis er auf Widerstand stößt.

FIG 1

FIG 2

FIG 3

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 967 129 (N.T. WINKLER) * Figur 1; Spalte 1, "Summary of the invention"; Spalte 2, Zeilen 29-41; Ansprüche 1,6,7 * | 1,4 | A 61 B 6/10 G 21 F 3/00 G 21 F 7/02 |
| A | --- | 3 | |
| Y | US-A-4 581 538 (H.J. LENHART) * Figur 1; Zusammenfassung; Spalte 2, Zeilen 43-50; Spalte 2, Zeilen 55-65; Ansprüche 1,8,10 * | 1,4 | |
| A | --- | 3 | |
| A | FR-A-1 125 061 (COMPAGNIE GENERALE DE RADIOLOGIE) * Anspruch; Figur 4 * | 2 | |
| A | DE-A-3 326 880 (K. KORTH) * Ansprüche 1,2; Seite 10, Zeilen 25-26; Seite 14, Zeilen 4-5 * ----- | 5 | |

**RECHERCHIERTE SACHGEBIETE (Int. Cl.5)**

A 61 B
G 21 F
G 21 K
H 05 G

Der vorliegende Recherchenbericht wurde für alle Patentansprüche erstellt

| Recherchenort | Abschlußdatum der Recherche | Prüfer |
|---|---|---|
| DEN HAAG | 22-11-1989 | FRITZ S.C. |